# EUROPEAN PATENT APPLICATION

(11) **EP 0 702 975 A1**
(43) Date of publication of application: **27.03.1996**
(21) Application number: 95202359.6
(22) Date of filing: 31.08.1995
(51) Int. Cl.: A61M 25/00, A61B 5/042

(54) **Catheter assembly and associated treatment catheter**

(30) Priority: 01.09.1994 NL 9401423
(71) Applicant: CORDIS EUROPA N.V., NL-9301 LJ Roden (NL)
(72) Inventor: Mous, Frans, NL-9302 HP Drachten (NL)
(74) Representative: 't Jong, Bastiaan Jacobus

(57) **Abstract**

The invention relates to a catheter assembly comprising a guiding catheter with a pliable tube-like basic body with a distal and a proximal end and a registration catheter slidable through a lumen of the guiding catheter which also has a tube-like basic body with a distal and a proximal end. In released state, the guiding catheter is bent at its distal end. The treatment catheter comprises at its distal end an end-section substantially shaped like part of a circle which, in released state, is positioned in a plane at right angles to the basic body. This end-section is provided with a number of electrodes which are connected to conductors extending through the basic body of the treatment catheter to its proximal end.

## Description

The invention relates to a catheter assembly to be used for cardiological purposes. It relates in particular to a catheter assembly for the registration of electrical impulses in the area surrounding the tricuspid valve in the right atrium of the heart of a patient.

When treating certain types of tachycardia it is advisable to measure the electrical activity inside the heart, and to ablate the area of the wall of the heart in question, when for instance an abnormality has been detected.

The invention relates to a catheter assembly with which a significant area surrounding the tricuspid valve on the atrial side can be treated.

Within the scope of this patent application the term treatment is understood to mean both diagnostic and interventional treatment, ie both the measurement of electrical activity and for instance the ablation of tissue.

With the catheter assembly of the invention as characterised in claim 1, the end-section of the treatment catheter which is substantially shaped like part of a circle, can be positioned, around the tricuspid valve, against the wall of the atrium and be used to carry out the required treatment.

The guiding catheter is first inserted into the body of the patient by means of a guide wire, whereby the curve at the distal end of the guiding catheter is advanced to a position above the tricuspid valve via the inferior vena cava. The end of the guiding catheter is directed towards the valve. Subsequently the treatment catheter is introduced via the guiding catheter. As soon as the end-section of the treatment catheter passes the end of the guiding catheter, it assumes its original shape and can be placed against the wall of the atrium surrounding the valve. In that position, the electrical activity can be registered or tissue ablated by means of the electrodes.

According to a further development the treatment catheter can be manufactured in such a way, that the centre of the end-section shaped like part of a circle is substantially placed on the continuation of the basic body. When the treatment catheter is turned inside the guiding catheter on its longitudinal axis, the end-section shaped like part of a circle will trace a circular path, so that a greater section or the entire circumference of the cardiac valve can be covered.

The embodiment of the treatment catheter as claimed in claim 7 is preferably employed for treatment of the area on the side of the valve where the interatrial septum is situated, and the embodiment according to claim 8 is preferably employed for treatment of the area on the side of the valve closest to the free wall of the heart.

The invention will be explained in greater detail in the following description with reference to the attached figures.

Fig. 1 represents a perspective view of a catheter assembly according to a first embodiment of the invention.

Fig. 2 shows a treatment catheter for a catheter assembly according to another embodiment.

Fig. 3 shows a cross-section of a heart with the catheter assembly of fig. 1 during treatment.

Fig. 4 represents the heart of fig. 3 in a partly broken away perspective view at an oblique angle seen from above, as indicated by arrow IV in fig. 3.

Fig. 5 shows a view corresponding to fig. 4 with the treatment catheter as shown in fig. 2.

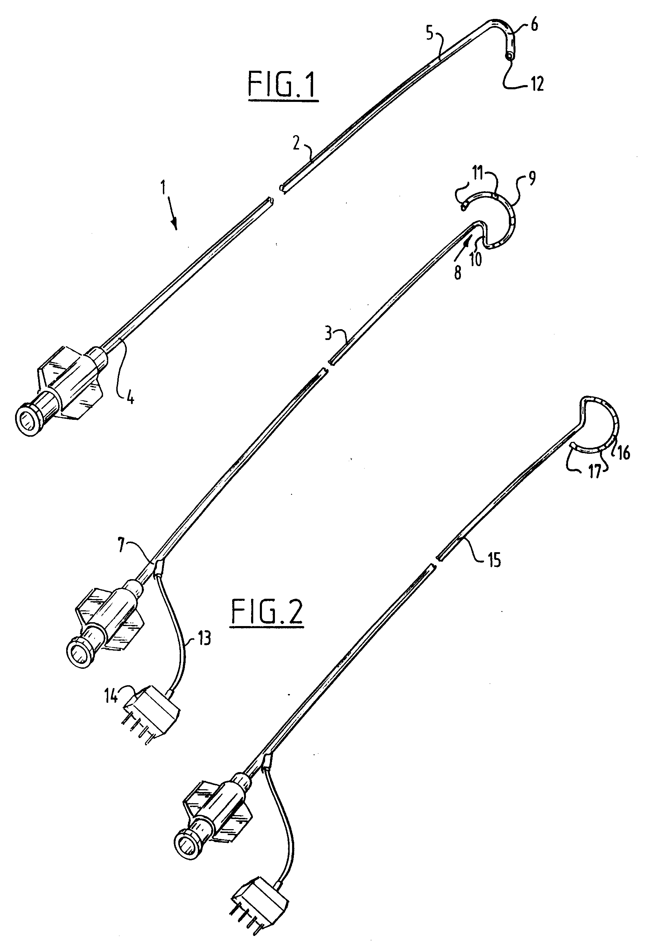

The catheter assembly 1 as shown in fig. 1 comprises a guiding catheter 2 and a treatment catheter 3.

In the usual manner, the guiding catheter 1 has a tube-like basic body with a proximal end and a distal end 5. At the proximal end a connecting member has been arranged.

At the distal end 5 the guiding catheter has a curve 6 which defines an angle of the order of 120 degrees.

On insertion, the guiding catheter 2 will be straightened at the curve 6 by means of a guide wire. Once the distal end 5 has arrived in the right atrium of the heart of a patient, the guide wire is removed and the distal end 5 will assume its original, bent shape.

The treatment catheter 3 has at its distal end 8 an end-section 9 which is substantially shaped like part of a circle. With the preferred embodiment as shown this end-section 9 traces an arch of substantially 180 degrees.

The end-section 9 shaped like part of a circle, is connected to the basic body by means of a connecting section 10. The distal end 8 has been preshaped in such a way that the end-section 9 is placed, in the released state of the treatment catheter 3, in a plane at right angles to the longitudinal axis of the basic body. As the catheter 3 has been made of a pliable material, the curved end can be straightened completely and introduced in this unbent form via the lumen 12 of the guiding catheter 2 into a patient.

In the preferred embodiment 4 shown, the end-section 9 shaped like part of a circle has been provided with a number of electrodes 11. These electrodes are connected to conductors 13 extending through the basic body of the catheter to its proximal end 7. These conductors 13 are connected to a connector 14 which in its turn is connected to a control means not specified here.

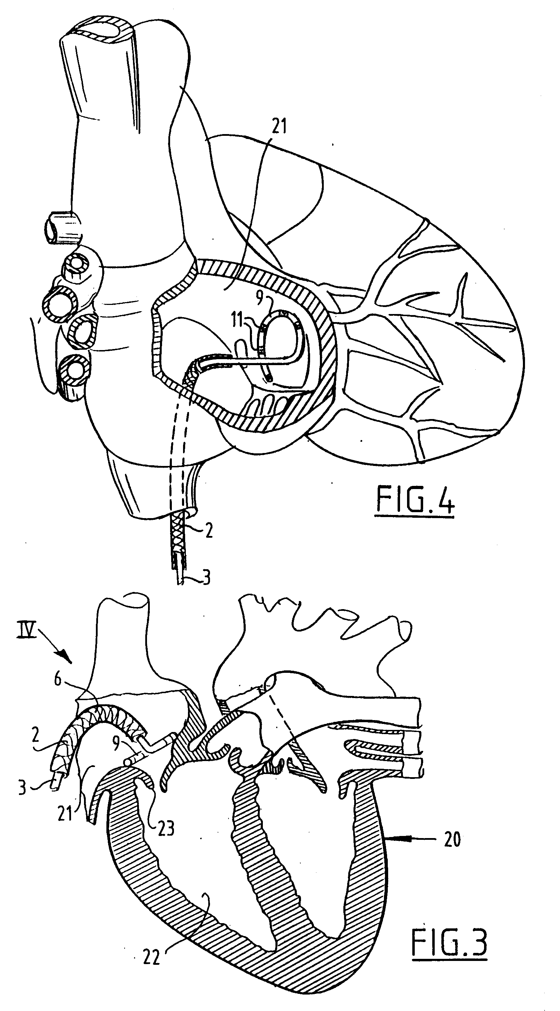

The treatment catheter 3 of fig. 1 comprises an end-section 9 which extends, as seen from the basic body, to the left. As will be explained in greater detail with reference to fig. 4, this embodiment is particularly designed for treatment of the atrial wall surrounding the tricuspid valve on the side of the interatrial septum.

The treatment catheter 15 of fig. 2 corresponds substantially to the treatment catheter 3 of fig. 1, whereby however the end-section 16 shaped like part of a circle extends, as seen from the basic body, to the right. This embodiment is particularly designed for treatment of the atrial wall surrounding the tricuspid valve on the side of the right atrial free wall.

The end-section 16 carries a number of electrodes 17 which, at the proximal end of the catheter, can be connected to a control device also by means of conductors.

Fig. 3 shows a catheter assembly according to the invention in the position of use.

Fig. 3 represents a cross-section of a heart 20 with a right atrium 21 and a right ventricle 22, with in between the tricuspid valve 23.

When using the catheter assembly according to the invention, the guiding catheter 2 is first introduced into the patient as described above in such a way, that its distal end is positioned inside the right atrium 21. On removal of the guide wire the curved end-section 6 of the guiding catheter will be released, so that the end of the guiding catheter 2 will be turned towards the centre of the valve 23.

Next the treatment catheter 3 is introduced via the lumen 12 of the guiding catheter 2. When the curved end-section has passed through the tip of the guiding catheter 2, it assumes the pre-bent shape, whereby the end-section 9 is placed in a plane at right angles to the basic body. As can be seen clearly in fig. 3, the end-section 9 can hereby be brought, around the tricuspid valve 23, into contact with the wall of the atrium 21. The electrodes 11 come into contact with this wall, so that the electrical activity can be measured and the tissue, if necessary, ablated.

The physician carrying out the procedure can optimize the contact between the end-section 9 shaped like part of a circle and the wall by rotating the proximal end of the catheter 3 a little. The circular end-section will also rotate in that case, whereby the basic body of the treatment catheter will function as a flexible axis.

As has been mentioned before, the end-section 9 shaped like part of a circle can be connected to the basic body in such a way that the centre of this end-section 9 shaped like part of a circle coincides with the continuation of the basic body. When rotating the basic body on its longitudinal axis, the end-section 9 can trace a circular path, so that in principle and dependent on the specific anatomy of the patient to be treated, a complete circle can be scanned.

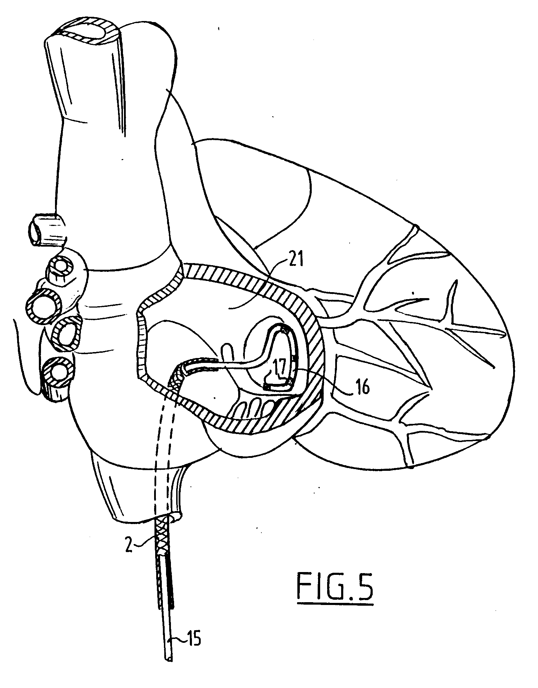

As can be seen in fig. 4 and 5 the two differently shaped treatment catheters 3 and 15 respectively are used for treatment of the atrial wall surrounding the tricuspid valve at the side of the interatrial septum and at the side of the right atrial free wall respectively. The shape of each of the two treatment catheters 3 and 15 has been adjusted in detail to the wall with which it has to make contact.

## Claims

1. Catheter assembly comprising a guiding catheter with a pliable tube-like basic body with a distal and a proximal end and a registration catheter slidable through a lumen of the guiding catheter which also has a tube-like basic body with a distal and a proximal end, wherein the guiding catheter is, in released state, curved at its distal end, and the treatment catheter comprises at its distal end an end-section substantially shaped like part of a circle which, in released state, is positioned in a plane at right angles to the basic body, which end-section is provided with a number of electrodes which are connected to conductors extending through the basic body of the treatment catheter to its proximal end.

2. Catheter assembly as claimed in claim 1, wherein the curve of the guiding catheter defines an angle between 90 and 150 degrees.

3. Catheter assembly as claimed in claim 2, wherein the curve of the guiding catheter defines an angle of at least 120 degrees.

4. Treatment catheter for a catheter assembly as claimed in one of the previous claims, comprising a tube-like basic body with a distal and a proximal end, wherein the treatment catheter comprises at its distal end an end-section substantially shaped like part of a circle which, in released state, is positioned in a plane at right angles to the basic body, which end-section is provided with a number of electrodes connected to conductors extending through the basic body of the treatment catheter to its proximal end.

5. Treatment catheter as claimed in claim 4, wherein the centre of the end-section shaped like part of a circle is situated on the continuation of the basic body.

6. Treatment catheter as claimed in claim 4 or 5, wherein the end-section shaped like part of a circle extends over an arc of substantially 180 degrees.

7. Treatment catheter as claimed in claim 6, wherein the end-section shaped like part of a circle extends, as seen from the basic body, to the left.

8. Treatment catheter as claimed in claim 6, wherein the end-section shaped like part of a circle extends, as seen from the basic body, to the right.
